# EUROPEAN PATENT APPLICATION

(11) **EP 4 205 784 A1**
(43) Date of publication of application: **05.07.2023**
(21) Application number: 20951589.9
(22) Date of filing: 31.08.2020
(51) Int. Cl.: A61M 5/315

(54) **PISTON**

(71) Applicant: Daikyo Seiko, LTD., Sano-shi, Tochigi 327-0813 (JP)
(72) Inventor: SUTO Hiroshi, Sano-shi, Tochigi 327-0813 (JP)
(74) Representative: Grünecker Patent- und Rechtsanwälte PartG mbB
(86) International application number: PCT/JP2020/032958
(87) International publication number: WO 2022/044340

(57) **Abstract**

Problem

To provide a piston for a syringe which can be easily formed by using a mold and can achieve both a high sealing performance and a low sliding resistance value.

Solution

A piston used by being inserted into a syringe barrel comprising an approximately cylindrical elastic body, the side surface includes plural annular protrusions in the axial direction, the maximum diameter part of the annular protrusions has an outer diameter which is to be contacted with the inner surface of the syringe barrel when the piston is inserted into the syringe barrel, and the maximum diameter part of at least one annular protrusion is formed at a position which is shifted from the halfway of the axial length of the annular protrusion toward the bottom surface side.

## Description

### Technical Field

The present invention relates to a piston which is suitable to use for a pharmaceutical or medical syringe, for example.

### Background Art

In general, a medical syringe comprises a syringe barrel having a tip on which a medicinal solution outlet is formed and a syringe plunger which is to be inserted into the syringe barrel from an opening on the other end for moving a piston in the axial direction. In a piston for a medical syringe it is required to have conflicting properties (performances), i.e., a high sealing performance and a high sliding performance with the inner surface of the syringe barrel, without interaction with a liquid for internal use (a medicinal solution) filled in the syringe barrel.

Especially in a piston for a prefilled syringe (serves as a container and syringe) in which a medicinal solution is filled in advance and which is being increasingly used lately, these properties are required at a higher level than a piston for a normal syringe, and it is required to be used safely over the long term without quality change, keep a high sealing performance (a high safety) for a high permeability medicinal solution and have a sufficient sliding performance such that an administration of a medicinal solution can be conducted smoothly.

### Prior Art Document

### Patent Document

Patent Document 1: Japanese laid-open patent application S57-22766
Patent Document 2: Japanese laid-open patent application 2006-181027

### Disclosure of Invention

### Problems to be resolved by the Invention

Even if a polytetrafluoroethylene (PTFE) film having a low friction coefficient is used on the surface of a piston, for example, a sufficient sliding performance is not necessarily obtained. Although it is known to set the compression ratio and the contact area to a value in a predetermined range as a method for achieving both a high sealing performance and a low sliding resistance value (Patent Document 1), a satisfactory result is not necessarily obtained. Although it is also known to achieve both a high sealing performance and a low sliding resistance value by forming plural ring-shaped protrusions continuously and integrally at the tip part of a piston(a gasket) (Patent Document 2), there is a problem that it is difficult to form thin ring-shaped protrusions by using a mold.

Therefore, it is desired to provide a piston for a syringe which is possible to be formed easily by using a mold and achieve both a high sealing performance and a low sliding resistance value.

### Means for solving the Problems

(1)A piston according to the present invention is formed by an approximately cylindrical shaped elastic body and is to be used by being inserted into a syringe barrel. The piston comprises an upper surface to be contacted with a liquid for internal use, a bottom surface with which a plunger rod is to be contacted and a side surface to be contacted with the inner surface of the syringe barrel when it is inserted into the syringe barrel. The side surface has plural annular protrusions in the axial direction, and the maximum diameter part of the annular protrusions has an outer diameter such that the maximum diameter part is to be contacted with the inner surface of the syringe barrel when it is inserted into the syringe barrel. In at least one annular protrusion, the maximum diameter part of the annular protrusion is formed at a position which is shifted from the halfway (a position which is 1/2 of the axial length) of the annular protrusion toward the bottom surface side.
   By this configuration, it is possible to provide a piston for a syringe which can achieve both a high sealing performance and a low sliding resistance value and can be easily formed by using a mold.
(2) In a piston according to one embodiment of the present invention, the side surface has a first annular protrusion, an annular recess and a second annular protrusion in this order from the upper surface side in the axial direction. The maximum diameter part of the first annular protrusion and the second annular protrusion has an outer diameter which is to be contacted with the inner surface of the syringe barrel when it is inserted into the syringe barrel. The maximum diameter part of the second annular protrusion is formed at a position which is shifted from the halfway (a position which is 1/2 of the axial length) of the second annular protrusion toward the bottom surface side.
   By this configuration, it is possible to provide a piston for a syringe which is suitable for being formed easily by using a mold and which can secure a high sealing performance mainly by the shape of the first annular protrusion and ensure a high sealing performance and reduce the sliding resistance value by the shape of the second annular protrusion, for example.
(3) In a piston according to the aforementioned (1) and (2), the side surface has a first annular protrusion, an annular recess and a second annular protrusion in this order from the upper surface side in the axial direction. The maximum diameter part of the first annular protrusion and the second annular protrusion has an outer diameter which is to be contacted with the inner surface of the syringe barrel when it is inserted into the syringe barrel, and the curvature radius of the second annular protrusion is set to be smaller than the curvature radius of the first annular protrusion.
   By this configuration, it is possible to provide a piston for a syringe which is suitable for being formed easily by using a mold and which can secure a high sealing performance mainly by the shape of the first annular protrusion and ensure a high sealing performance and reduce the sliding resistance value preferably by the shape of the second annular protrusion, for example.
(4) In a piston according to the aforementioned (2) and (3), the tilt angle of the surface headed for the maximum diameter part of the second annular protrusion from the annular recess relative to the annular recess is set to be smaller than the tilt angle of the surface headed for the bottom surface side from the maximum diameter part relative to the annular recess.
   By this configuration, it is possible to provide a piston for a syringe having a second annular protrusion which can be formed easily by using a mold and which can achieve both a high sealing performance and a low sliding resistance value.
(5) Additionally in a piston according to either one of the aforementioned (2) through (4), the contact area of the second annular protrusion to be contacted with the inner surface of the syringe barrel is set to be smaller than the contact area of the first annular protrusion to be contacted with the inner surface of the syringe barrel when it is inserted into the syringe barrel.
   By this configuration, it is possible to provide a piston for a syringe which can be formed easily by using a mold and which can secure a high sealing performance by the shape of the first annular protrusion and reduce the sliding resistance value by the shape of the second annular protrusion.

### Effect of Invention

According to the present invention, it is possible to provide a piston which can reduce the sliding resistance value and prevent the liquid leakage by securing a high sealing performance.

### Brief Description of Drawings

Fig. 1 shows a schematic view of a configuration of a piston according to one embodiment of the present invention, and the right half portion shows a front view and the left half portion shows a cross-sectional view.
Fig.2 shows an enlarged view of the right edge part of the piston shown in Fig.1.
Fig.3 (A) shows the right half portion of the piston shown in Fig.1, and (B)shows an enlarged view of the portion (B) shown by the dotted line in (A).
Fig. 4 (A) shows a schematic view of a configuration of a piston according to a comparative example, the right half portion shows a front view and the left half portion shows a cross-sectional view, and (B) is an enlarged view showing the shape of the right edge part of the piston shown in (A).
Fig. 5 (A) shows the right edge part of the piston according to the embodiment shown in Fig. 1, and (B) shows an enlarged view of the portion (B) shown by the dotted line in (A).
Fig. 6 (A) shows the right edge part of the piston according to the comparative example shown in Fig. 4, and (B) shows an enlarged view of the portion (B) shown by the dotted line in (A).
Fig. 7 (A) is a schematic view showing a condition in which the piston according to the embodiment shown in Fig. 1 is inserted into a syringe barrel, and (B) is a schematic view showing a condition in which the piston according to the comparative example shown in Fig. 4 is inserted into a syringe barrel.
Fig. 8 (A) shows a test result of measuring the sliding resistance value one day after the piston according to the embodiment shown in Fig. 1 was inserted into a syringe barrel, and (B) shows a test result of measuring the sliding resistance value one day after the piston according to the comparative example shown in Fig. 4 was inserted into a syringe barrel.
Fig. 9 (A) shows a test result of measuring the sliding resistance value one month after the piston according to the embodiment shown in Fig. 1 was inserted into a syringe barrel, (B) shows a test result of measuring the sliding resistance value one month after the piston according to the comparative example shown in Fig. 4 was inserted into a syringe barrel.
Fig. 10 (A) is a pattern diagram showing the shape change before sliding and during sliding of the second annular protrusion in the piston according to the embodiment shown in Fig. 1, and (B) is a pattern diagram showing the shape change before sliding and during sliding of the second annular protrusion in the piston according to the comparative example shown in Fig.4.
Fig. 11 (A) through (F) are schematic views showing the shapes of the right edge parts of pistons according to other embodiments of the present invention.

### A Mode for implementing the Invention

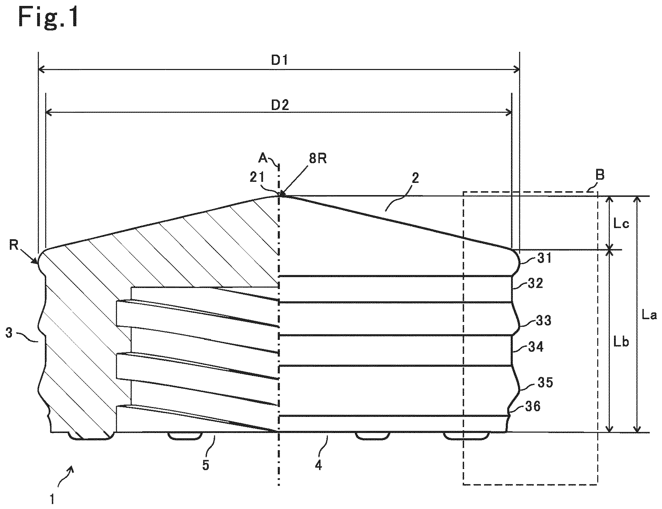

A configuration of a piston according to one embodiment of the present invention will be described below referring to the drawings. Fig. 1 shows a configuration of a piston according to one embodiment of the present invention. A piston 1 is an approximately cylindrical molded body made of an elastic material. It is preferable to use an elastic material or a flexible material as a material used for a piston constituting a syringe.

As an elastic material, it is possible to use a synthetic rubber, for example, a compound which combines a main raw material with a filler material, a crosslinking agent and so on. The main raw material can be selected from isobutylene-isoprene rubber (IIR), chlorinated isobutylene-isoprene rubber (CIIR), brominated isobutylene-isoprene rubber (BIIR), partial cross-linkage IIR, polybutadiene rubber(BR), polyisoprene rubber (IR), ethylene-propylene-diene terpolymerization rubber (EPDM), styrene-butadiene copolymerization rubber (SBR), acrylic rubber (ACM), acrylonitrile butadiene rubber(NBR), etc. Among other things, it is preferable to use isobutylene-isoprene rubber (IIR), chlorinated isobutylene-isoprene rubber (CIIR) and brominated isobutylene-isoprene rubber (BIIR) from the perspective of gas barrier properties and elution characteristics, etc.

It is also possible to use a thermoplastic elastomer as an elastic material. For example, it is preferable to use a species alone or a mixture of two or more species selected from olefinic system(TPO), styrene system (SBC), vinyl chloride system(TPVC), urethane system (TPU), polyester system (TPEE), polyamide system (TPAE), fluorine system (TPF), polybutadiene system (RB), polyisobutylene system, silicone system, ethylene-vinyl acetate system (EVA,EEA), polyisobutylene thermoplastic elastomer (SIBS), and styrene-based elastomer such as styrene-butadiene-styrene (SBS) copolymer, styrene-ethylene butylene-styrene (SEBS) copolymer, styrene-isoprene- styrene (SIS) copolymer, etc., as well as ethylene-propylene non-conjugated diene monomeric (EPDM) copolymer, ethylene-propylene (EPM)copolymer and so on.

Among other things, it is preferable to use styrene-ethylene butylene-styrene (SEBS) copolymer, styrene-butadiene-styrene (SBS) copolymer, styrene-isoprene (SIS) copolymer, styrene-isobutylene (SIBS) copolymer, etc., in view of the heat resistance properties, elution characteristics, etc.

As a flexible material, it is preferable to use a species alone or a mixture of two or more species selected from thermoplastic resin such as PE resin, PP resin, PC resin, ABS resin, polyamide resin, polyester resin, etc., for example.

The piston 1 has a rotationally symmetric outer shape centered on the central axis A shown by a dashed-dotted line, and in Fig.1, the right side of the central axis A is shown in a front view and the left side is shown in a cross-sectional view.

The piston 1 having an axial length La is formed such that it has an outer shape of a cylinder having an axial length Lb in which one of the end surfaces is connected to the bottom surface of a cone having an axial length Lc. The side surface of the cone corresponds to the upper surface 2 of the piston 1, and this shape is preferably matched with the shape of the inner surface of the tip of a syringe barrel into which the piston 1 is inserted. Although the upper surface 2 may be the side surface of a complete cone, it is also possible to form it such that the vicinity of the vertex 21 is rounded.

On the side surface 3 of the piston 1, a first annular protrusion 31, a first annular recess 32, a second annular protrusion 33, a second annular recess 34, a third annular protrusion 35 and a third annular recess 36 are formed in this order from the side of the upper surface 2. The third annular recess 36 is formed such that it continues to the bottom surface 4 which is the other end surface of the cylinder. A screw hole 5 is formed in the center of the bottom surface 4, and it is to be screwed with a screw thread at the tip portion of a plunger rod which is not shown in the drawings.

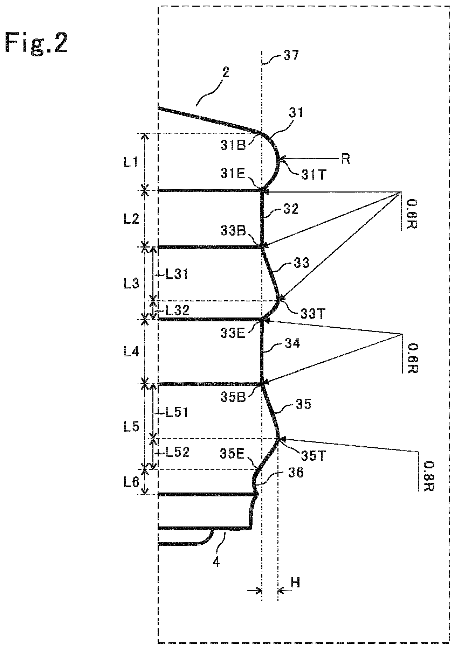

Fig. 2 shows an enlarged view of a portion B surrounded by a dotted line in Fig.1. In Fig.2, the outer diameter at the vertex of each of the first annular protrusion 31, the second annular protrusion 33 and the third annular protrusion 35 protrusion, namely the maximum outer diameter of each of the first annular protrusion 31, the second annular protrusion 33 and the third annular protrusion 35 is set to an equal value. This maximum outer diameter corresponds to the maximum outer diameter D1of the cylinder shown in Fig.1. The depth of the first annular recess 32 and the depth of the second annular recess 34, namely the difference between the bottom surface of the first annular recess 32 and the maximum diameter and the difference between the bottom surface of the second annular recess 34 and the maximum diameter are set to an equal value. On the side surface 3 of the piston 1, each of the first annular protrusion 31, the second annular protrusion 33 and the third annular protrusion 35 protrudes by a height H respectively from an extended bottom surface 37 which is extend as if the bottom surfaces of the two annular recesses 32 and 34 are connected. The outer diameter of the portion surrounded by the extended bottom surface 37 is D2.

The axial cross-sectional shape of the side surface 3 of the piston 1 will be explained in the following. The first annular protrusion 31 starts at the start point 31B which is the intersection between the upper surface 2 and the extended bottom surface 37, reaches the extended bottom surface 37 at a position which is lower than the start point 31B by a length L1 in the axial direction and which corresponds to the end point 31E of the first annular protrusion 31. The axial cross-sectional shape of the first annular protrusion 31 is arc shaped having the center inside the piston 1 except for the vicinity of the start point 31B and the vicinity of the end point 31E, the arc is a part of one circle or a continuous connection of parts of plural circles each having a different radius. The vicinity of the end point 31E has an arc shape having the center outside the piston 1.

The start point 33B of the second annular protrusion 33 is positioned on the extended bottom surface 37 with an interval of an axial length L2 of the first annular recess 32 from the end point 31E, the second annular protrusion 33 reaches the extended bottom surface 37 at a position lower than the start point 33B by a length L3 in the axial direction, and the reached position corresponds to the end point 33E of the second annular protrusion 33. The axial cross-sectional shape of the second annular protrusion 33 is approximately linear shaped except for the vicinity of the start point 33B, the vicinity of the end point 33E and the vicinity of the vertex 33T, the part beyond the vertex 33T is arc shaped having the center inside the piston 1 except for the vicinity of the end point 33E, and the arc is a part of one circle or a contentious connection of parts of plural circles each having a different radius. The vicinity of the start point 33B and the vicinity of the end point 33E are arc shaped having the center outside the piston 1.

The start point 35B of the third annular protrusion 35 is positioned on the extended bottom surface 37 with an interval of an axial length L4 of the second annular recess 34 from the end point 33E, the third annular protrusion 35 reaches the extended bottom surface 37 at a position lower than the start point 35B by a length L5 in the axial direction, and the reached position corresponds to the end point 35E of the third annular protrusion 35. The third annular recess 36 has an axial length L6 and the piston has a configuration which reaches the vicinity of the bottom surface 4 from the end point 35 E through the third annular recess 36. The axial cross-sectional shape of the third annular protrusion 35 is approximately linear shaped except for the vicinity of the start point 35B and the vicinity of the vertex 35T, the part beyond the vertex 35T is arc shaped having the center inside the piston 1 except for the vicinity of the end point 35E, and the arc is a part of one circle or a contentious connection of parts of plural circles each having a different radius. The vicinity of the start point 35B and the vicinity of the end point 35E are arc shaped having the center outside the piston 1.

Next, the axial cross-sectional shape of the side surface 3 of the piston 1 will be explained. In Fig. 2, supposing that the curvature radius of the vicinity of the vertex 31T of the first annular protrusion 31 is R, the curvature radius of the vicinity of the vertex 33T of the second annular protrusion 33 is 0.6R and it is smaller than the curvature radius R of the vicinity of the vertex 31T of the first annular protrusion 31. The curvature radius of the vicinity of the vertex 35T of the third annular protrusion 35 is 0.8R and it is smaller than the curvature radius R of the vicinity of the vertex 31T of the first annular protrusion 31.

The curvature radius of the vicinity of each of the end point 31E, the start point 33B, the end point 33E and the start point 35B is 0.6R, and the first annular protrusion 31, the first annular recess 32, the second annular protrusion 33, the second annular recess 34 and the third annular protrusion 35 are connected smoothly and continuously. The curvature radius of the vicinity of the vertex 21 of the upper surface 2 shown in Fig.1 is 8R.

In Fig.2, the axial length of the first annual protrusion 31 between the start point 31B and the end point 31E is L1, the axial length of the first annual recess 32 is L2, the axial length of the second annual protrusion 33 between the start point 33B and the end point 33E is L3, the axial length of the second annual recess 34 is L4, the axial length of the third annual protrusion 35between the start point 35B and the end point 35E is L5, and the axial length of the third annual recess 36 is L6.

The axial length L31 of the second annual protrusion 33 between the start point 33B and the vertex 33T is longer than the axial length L32 between the vertex 33T and the end point 33E. The axial length L51 of the third annual protrusion 35 between the start point 35B and the vertex 35Tis longer than the axial length L52 between the vertex 35T and the end point 35E. In this way, the vertex (maximum diameter part) 33T of the second annual protrusion 33 is shifted from the halfway (the position which is 1/2 of the axial length from the start point or the end point) of the second annular protrusion 33 toward the side of the bottom surface 4. The vertex (maximum diameter part) 35T of the third annual protrusion 35 is shifted from the halfway (the position which is 1/2 of the axial length from the start point or the end point) of the third annular protrusion 35 toward the side of the bottom surface 4.

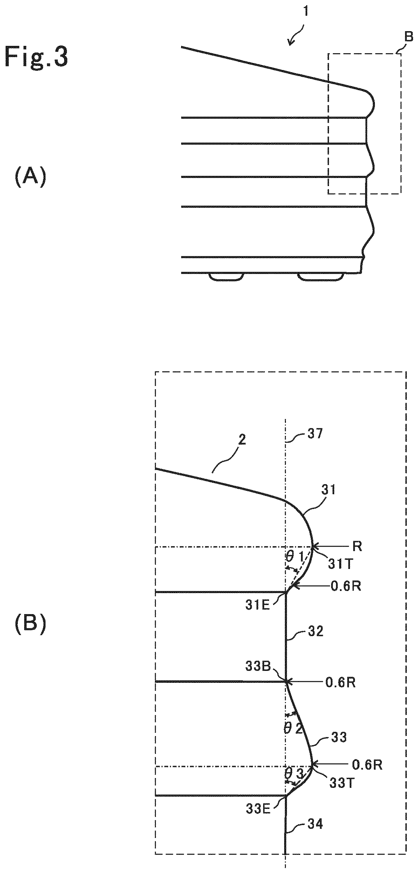

Fig. 3(B) is an enlarged view of the portion B surrounded by a dotted line in Fig. 3(A) which shows a part of the piston 1. In Fig. 3(B), the angle of a line (shown in the drawing by a dotted line) connecting the vertex 31T with the end point 31E of the first annular protrusion 31 relative to the extended bottom surface 37 is *θ* 1. The angle of the line (shown in the drawing by a dotted line, however it is mostly overlapped with the curved surface of the cross section of the second annular protrusion 33 which is almost linear) connecting the start point 33B with the vertex 33T of the second annular protrusion 33 relative to the extended bottom surface 37 is *θ* 2. The angle of the line (shown in the drawing by the dotted line) connecting the vertex 33T with the end point 33E of the second annular protrusion 33 relative to the extended bottom surface 37 is *θ* 3.

In this case, the angle *θ* 2 is set to be smaller than the angle *θ* 1, and the tilt angle of the surface between the start point 33B and the vertex 33T of the second annular protrusion 33 relative to the extended bottom surface 37 is smaller than the tilt angle of the surface between the vertex 31T and the end point 31E of the first annular protrusion 31 relative to the extended bottom surface 37. The angle *θ* 2 is set to be smaller than the angle *θ* 3, and the tilt angle of the surface between the start point 33B and the vertex 33T of the second annular protrusion 33 relative to the extended bottom surface 37 is smaller than the tilt angle of the surface between the vertex 33T and the end point 33E of the second annular protrusion 33 relative to the extended bottom surface 37.

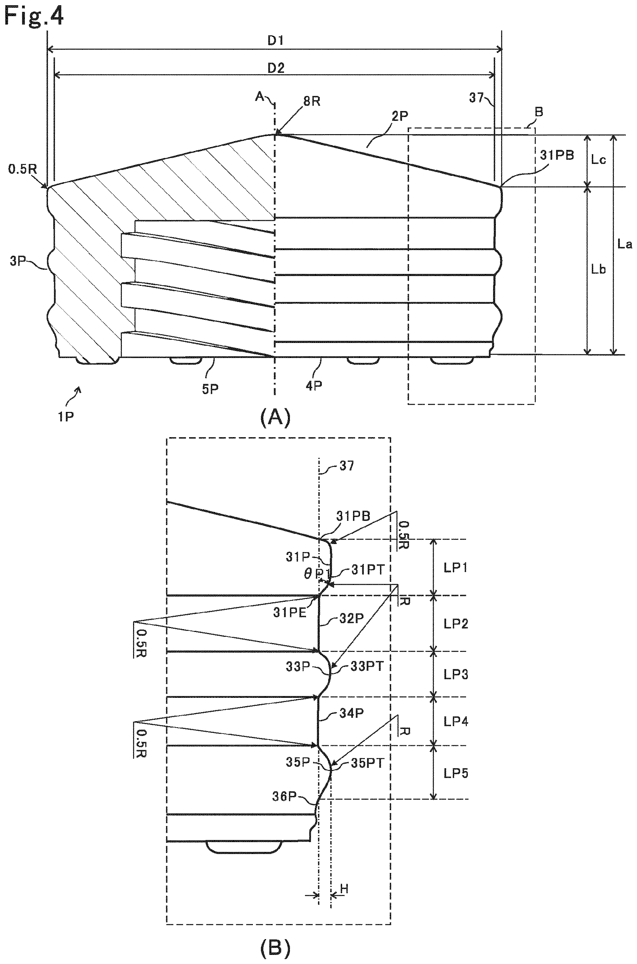

Fig. 4 shows a configuration of the piston 1P according to a comparative example. In Fig. 4(A), the piston 1P has a rotationally symmetric outer shape centered on the central axis A shown by a dashed-dotted line, and the right side of the central axis A is shown in a front view and the left side is shown in a cross-sectional view in Fig.4(A). The piston 1P is formed such that it has an outer shape of a cylinder in which one of the end surfaces is connected to the bottom surface of a cone, similar to the piston 1 shown in Fig.1.

The shapes of these side surfaces 3 and 3P are different, however the axial length of the entire piston La, the axial length of the cylinder part Lb, the axial length of the cone part Lc, the maximum outer diameter D1 and the outer diameter D2 of the extended bottom surface 37 of the annular recess are equal respectively between the piston 1 in Fig. 1 and the piston 1P in Fig. 4(A).

Fig. 4(B) shows an enlarged view of the side surface 3P of the piston 1P. A first annular protrusion 31P, a first annular recess 32P, a second annular protrusion 33P, a second annular recess 34P, a third annular protrusion 35P and a third annular recess 36P are formed in this order on the side surface 3P from the upper surface 2P toward the bottom surface 4P.

The outer diameter of each vertex of the first annular protrusion 31P, the second annular protrusion 33P and the third annular protrusion 35P, namely the maximum outer diameter of each of the first annular protrusion 31P, the second annular protrusion 33P and the third annular protrusion 35P is set to an equal value. The depth of the first annular recess 32P and the depth of the second annular recess 34P, namely the difference between the bottom surface of the annular recess 32P and the maximum diameter and the difference between the bottom surface of the annular recess 34P and the maximum diameter are set to an equal value. On the side surface 3P of the piston 1P, the first annular protrusion 31P, the second annular protrusion 33P and the third annular protrusion 35P protrudes by the height H respectively from the extended bottom surface 37 which is extended such that the bottom surfaces of the two annular recesses 32P and 34P are connected.

The intersection between the upper surface 2P and the extended bottom surface 37 corresponds to the start point 31PB of the first annular protrusion 31P, and the side surface 3P reaches the extended bottom surface 37 at a position which is lower than the start point 31PB by a length LP1 in the axial direction and the reached position corresponds to the end point 31PE of the first annular protrusion 31P. The vertex 31PT of the first annular protrusion 31P has a flat part, namely the curvature radius at the vertex 31PT and in its vicinity is infinite or extremely large. The curvature radius of the curved surface continuing from the start point 31PB to the flat part is 0.5R, the curvature radius of the curved surface continuing from the flat part toward the first annular recess 32P is R, and the curvature radius of the curved surface in the vicinity of the end point 31PE is 0.5R.

The start point 33PB of the second annular protrusion 33P is positioned on the extended bottom surface 37 at an interval of an axial length LP2 of the first annual recess 32P from the end point 31PE. The second annular protrusion 33P has a vertically symmetrical arc shaped cross-sectional curved surface about the axis of symmetry which is the horizontal line from the vertex 33PT toward the central axis of the piston. The curved surface reaches the extended bottom surface 37 at a lower position than the start point 33PB by a length LP3 in the axial direction, and the reached position corresponds to the end point 33PE of the second annular protrusion 33P. The curvature radius of the vicinity of the start point 33PB is 0.5R, the curvature radius of the vicinity of the vertex 33PT is R, and the curvature radius of the vicinity of the end point 33PE is 0.5R.

The start point 35PB of the third annular protrusion 35P is positioned on the extended bottom surface 37 at an interval of an axial length LP4 of the second annual recess 34P from the end point 33PE, the curved surface reaches the extended bottom surface 37 at a lower position than the start point 35PB by a length LP5 in the axial direction, and the reached position corresponds to the end point 35PE of the third annular protrusion 35P. The curvature radius of the vicinity of the start point 35PB is 0.5R, and the curvature radius of the vicinity of the vertex 35PT is R.

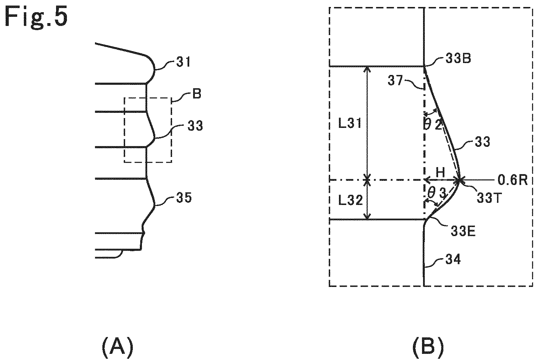

Fig. 5(A) shows the right edge part of the piston 1 according to one embodiment of the present invention, and Fig.5 (B) shows an enlarged view of the portion B of the second annular protrusion 33 shown by the dotted line in Fig. 5(A). In Fig. 5(B), the axial length of the second annular protrusion 33 of the piston 1 according to one embodiment of the present invention is L3, and the axial length L31 from the start point 33B to the vertex 33T is set to be longer than the axial length L32 from the vertex 33T to the end point 33E. In other ward, the maximum diameter part (the vertex 33T) of the second annular protrusion 33 is formed at a position which is shifted from the halfway (a position which is 1/2 of the axial length) of the second annular protrusion 33 toward the side of the bottom surface 4.

The angle of the line (shown in the drawing by the dotted line) connecting the start point 33B with the vertex 33T of the second annular protrusion 33 relative to the extended bottom surface 37 is *θ* 2, and the angle of the line (shown in the drawing by the dotted line) connecting the vertex 33T with the end point 33E relative to the extended bottom surface 37 is *θ* 3. The angle *θ* 2 is set to be smaller than the angle *θ* 3, the tilt angle of the curved surface from the start point 33B to the vertex 33T is smaller than the tilt angle of the curved surface from the vertex 33T to the end point 33E. The curvature radius of either one of the vicinity of the start point 33B, the vicinity of the vertex 33T and the vicinity of the end point 33E is 0.6R.

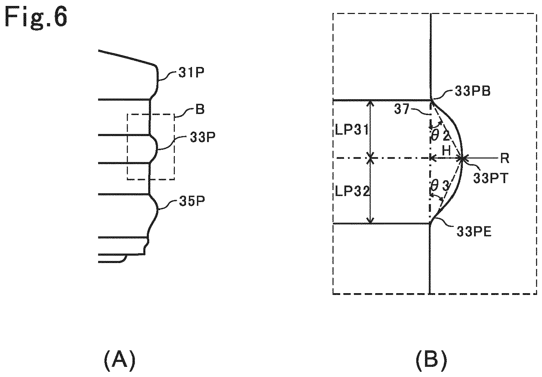

Fig. 6(A) shows the right edge part of the piston 1P according to the comparative example shown in Fig.4, and Fig.6(B) shows an enlarged view of the portion B of the second annular protrusion 33P shown by the dotted line in Fi.6(A). In Fig. 6(B), the axial length of the second annular protrusion 33P of the piston 1P according to the comparative example is LP3, and the axial length LP31 from the start point 33PB to the vertex 33PT is equal to the axial length LP32 from the vertex 33PT to the end point 33PE.

As shown in Fig.4(B), the angle of the line connecting the vertex 31PT, which forms the maximum outer diameter part of the first annular protrusion 31P and is positioned nearest to the bottom surface side, with the end point 31PE relative to the extended bottom surface 37 is *θ* P1. In Fig.6 (B), the angle of the line (shown in the drawing by the dotted line) connecting the start point 33PB with the vertex 33PT relative to the extended bottom surface 37 is *θ* P2, and the angle of the line (shown in the drawing by the dotted line) connecting the vertex 33PT with the end point 33PE relative to the extended bottom surface 37 is *θ* P3. The angle *θ* P2 is set to be smaller than the angle *θ* P1, and the angle *θ* P2 is set to be equal to the angle *θ* P3. In other ward, the shape of the curved surface from the start point 33PB to the vertex 33PT is identical to the shape of the curved surface from the end point 33PE to the vertex 33PT. The curvature radius at the vicinity of the start point 33PB and the vicinity of the end point 33PE is 0.5R, and the curvature radius at the vicinity of the vertex 33PT is R.

The curvature radius at the vertex 33T of the second annular protrusion 33 according to the present embodiment is 0.6R, and it is set to be smaller than the curvature radius R at the vertex 33PT of the second annular protrusion 33P according to the comparative example. The angle *θ* 2 of the line connecting the start point 33B with the vertex 33T relative to the extended bottom surface 37 is set to be smaller than the angle *θ* P2 of the line connecting the start point 33PB with the vertex 33PT relative to the extended bottom surface 37.

In the second annular protrusion 33 according to the present embodiment, the curved surface intersects with the extended bottom surface 37 at the start point 33B above the vertex 33T, and the curved surface intersects with the extended bottom surface 37 at the end point 33E below the vertex 33T. The distance between the start point 33B and the vertex 33T is different from the distance between the vertex 33T and the end point 33E, and the second annular protrusion 33 has an above-below asymmetric shape about the axis which is the horizontal line extending from the vertex 33T to the central axis of the piston. On the other hand, in the second annular protrusion 33P according to the comparative example, the curved surface intersects with the extended bottom surface 37 at the start point 33PB above the vertex 33PT, and the curved surface intersects with the extended bottom surface 37 at the end point 33PE below the vertex 33PT. The distance between the start point 33PBand the vertex 33PT is equal to the distance between the vertex 33PT and the end point 33PE, and the second annular protrusion 33P has an above-below symmetric shape.

As shown in Fig.5(B) and Fig.6(B), while the curved surface from the vicinity of the start point 33B up to the vertex 33T of the second annular protrusion 33 is a moderate slope in the present embodiment, the curved surface from the vicinity of the start point 33PB up to the vertex 33PT of the second annular protrusion 33P is a slope standing up with a relatively steep gradient in the comparative example.

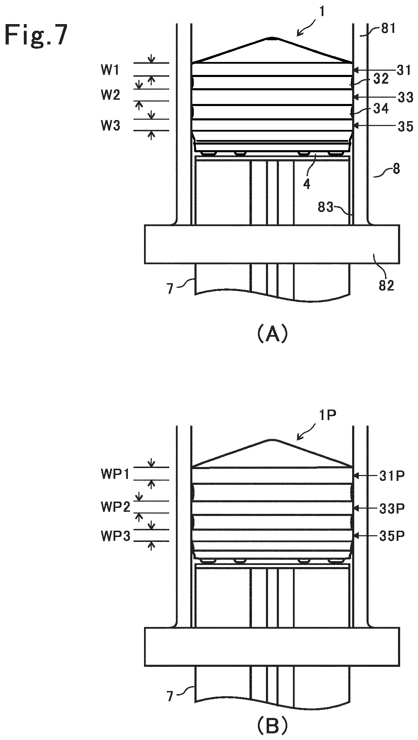

Fig. 7(A) shows a condition in which the piston 1 is pushed into a cylinder part 81 of a syringe barrel 8, which is formed by glass or plastics, from the opening of a flange part 82, after connecting a plunger rod 7 with the piston 1 by screwing the plunger rod 7, which has a tip with screw threads, into a screw hole 5 of the piston 1 according to one embodiment of the present invention which is explained referring to Fig.1 through Fig.3. The inner diameter of the cylinder part 81 is set to be slightly smaller than the maximum outer diameter of the piston 1, and the first annular protrusion 31, the second annular protrusion 33 and the third annular protrusion 35 are pushed against the inner surface 83, and then the vertex 31T, the vertex 33T and the vertex 35T are to be in a condition in which they are slightly deformed.

Fig. 7(B) shows a condition in which the piston 1P is pushed into the cylinder part 81 of the syringe barrel 8, which is formed by glass or plastics, from the opening of the flange part 82, after connecting the plunger rod 7 with the piston 1P by screwing the plunger rod 7, which has a tip with screw threads, into a screw hole 5P of the piston 1P according to the comparative example which is explained referring to Fig.4. The inner diameter of the cylinder part 81 is set to be slightly smaller than the maximum outer diameter of the piston 1P, and the first annular protrusion 31P, the second annular protrusion 33P and the third annular protrusion 35P are pushed against the inner surface 83, and then the vertex 31PT, the vertex 33PT and the vertex 35PT are to be in a condition in which they are slightly deformed.

The maximum outer diameter of the piston 1 is set to be equal to the maximum outer diameter of the piston 1P. In other word, each maximum outer diameter of the first annular protrusion 31, the second annular protrusion 33 and the third annular protrusion 35 is set to be equal to each maximum outer diameter of the first annular protrusion 31P, the second annular protrusion 33P and the third annular protrusion 35P. The inner diameter of the cylinder part 81 in the syringe barrel 8 in Fig. 7 (A) is set to be equal to the inner diameter of the cylinder part 81 in the syringe barrel 8 in Fig.7(B).

Table 1 shows a result of measuring the widths of the parts contacting with the inner surface 83 of the syringe barrel 8 with respect to the piston 1 according to the present embodiment and the piston 1P according to the comparative example. For the piston 1, the width W1 of the part in which the first annular protrusion 31 contacts with the inner surface 83, the width W2 of the part in which the second annular protrusion 33 contacts with the inner surface 83 and the width W3 of the part in which the third annular protrusion 35 contacts with the inner surface 83 are measured at the left edge and the right edge of the cylinder part 81 of the syringe barrel 8 shown in Fig.7(A).

For the piston 1P, the width WP1 of the part in which the first annular protrusion 31P contacts with the inner surface 83, the width WP2 of the part in which the second annular protrusion 33P contacts with the inner surface 83 and the width WP3 of the part in which the third annular protrusion 35P contacts with the inner surface 83 are measured at the left edge and the right edge of the cylinder part 81 of the syringe barrel 8 shown in Fig.7(B).

**Table 1**

| | Left edge | Right edge | Average | | Left edge | Right edge | Average |
|---|---|---|---|---|---|---|---|
| W1 | 0.90mm | 0.89mm | 0.90mm | WP1 | 1.62mm | 1.60mm | 1.61mm |
| W2 | 0.51mm | 0.51mm | 0.51mm | WP2 | 0.54mm | 0.56mm | 0.55mm |
| W3 | 0.50mm | 0.52mm | 0.51mm | WP3 | 0.55mm | 0.57mm | 0.56mm |

As shown in Table 1, in the piston 1 according to the present embodiment, the contact area (proportional to the width W2), in which the second annular protrusion 33 contacts with the inner surface 83 of the syringe barrel 8, is smaller than the contact area (proportional to the width W1), in which the first annular protrusion 31 contacts with the inner surface 83 of the syringe barrel 8. The contact area (proportional to the width W3), in which the third annular protrusion 35 contacts with the inner surface 83 of the syringe barrel 8, is smaller than the contact area (proportional to the width W1), in which the first annular protrusion 31 contacts with the inner surface 83 of the syringe barrel 8.

The contact area (proportional to the width W1), in which the first annular protrusion 31 contacts with the inner surface 83 of the syringe barrel 8, in the piston 1 according to the present embodiment is smaller than the contact area (proportional to the width WP1), in which the first annular protrusion 31P contacts with the inner surface 83 of the syringe barrel 8 in the piston 1P according to the comparative example. The contact area (proportional to the width W2), in which the second annular protrusion 33 contacts with the inner surface 83 of the syringe barrel 8, in the piston 1 according to the present embodiment is smaller than the contact area (proportional to the width WP2), in which the second annular protrusion 33P contacts with the inner surface 83 of the syringe barrel 8 in the piston 1P according to the comparative example. The contact area (proportional to the width W3), in which the third annular protrusion 35 contacts with the inner surface 83 of the syringe barrel 8, in the piston 1 according to the present embodiment is smaller than the contact area (proportional to the width WP3), in which the third annular protrusion 35P contacts with the inner surface 83 of the syringe barrel 8 in the piston 1P according to the comparative example.

The general material properties required for a medical piston is to have a low dissolvability, a low hydrous property and a high barrier property. It is preferable for an elastic body used for the piston 1 to have a hardness of 40 through 70 Shore-A hardness according to JISK6253-3(2012). It is also preferable to have a compression set according to JISK6262(2013) of 40% or less and it is more preferable to have a compression set of 3% or more and 40% or less.

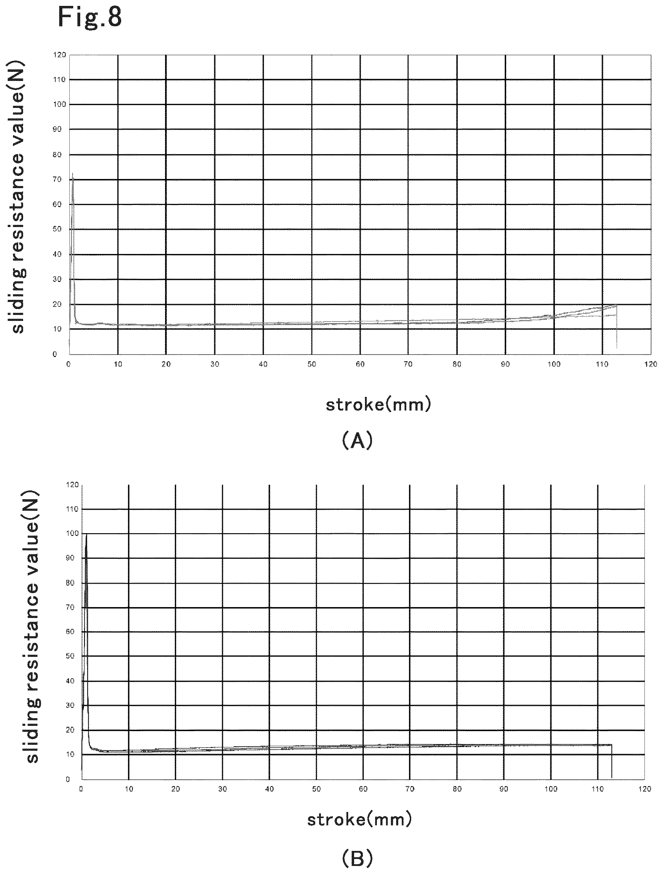

As shown in Fig. 7(A),(B), each of the non-laminated rubber pistons 1 and 1P was assembled with the syringe barrel 8, in which the inner surface 83 is coated by silicone oil, as a syringe of 100mL respectively. Tables 2 and 3 each show a result of the test, in which each piston was pushed toward the tip one day after the assembly using water as a liquid for internal use, conducted using a universal resting instrument "Autograph" made by Shimadzu Corporation. The measurement result for the piston 1 according to the present embodiment is shown in Fig.8(A) and the measurement result for the piston 1P according to the comparative example is shown in Fig.8(B), in which the horizontal axis is the stroke(mm) and the vertical axis is the sliding resistance value (N).

**Table 2 sliding resistance value test, one day after assembly (present embodiment)**

| Sample | Average | Maximum value | Minimum value |
|---|---|---|---|
| 1 | 23.52N | 72.63N | 11.67N |
| 2 | 22.90N | 71.09N | 11.50N |
| 3 | 23.14N | 71.18N | 11.99N |

**Table 3 sliding resistance value test, one day after assembly (comparative example)**

| Sample | Average | Maximum value | Minimum value |
|---|---|---|---|
| 1 | 27.43N | 95.81N | 12.64N |
| 2 | 27.42N | 98.06N | 11.83N |
| 3 | 27.29N | 99.98N | 11.37N |

In Fig.8(A),(B), the vicinity of a point, where the stroke is 0mm, corresponds to the beginning of the movement of each of the pistons 1 and 1P, where each measurement result shows the maximum sliding resistance value. And then the sliding resistance value decreases rapidly, and it shows the minimum sliding resistance value when each of the pistons 1 and 1P starts moving. Each of the pistons 1 and 1P was pushed in the syringe barrel 8 up to the predetermined position by moving toward the tip of the syringe barrel. As shown in Fig.8(A),(B), Table 2 and Table 3, both the average value and the maximum value of the sliding resistance in the piston 1 according to the present embodiment is more reduced than those in the piston 1P according to the comparative example.

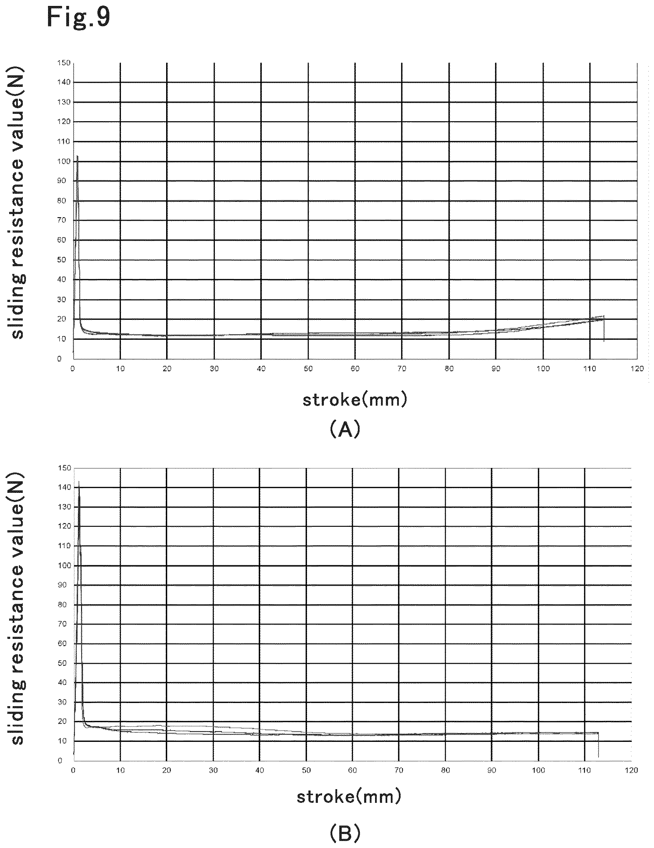

Similar to the tests shown in Table 2, Table 3, and Fig.8 (A), (B), each of the non-laminated rubber pistons 1 and 1P was assembled with the syringe barrel 8, in which the inner surface 83 is coated by silicone oil, as a syringe of 100mL respectively. Each piston was pushed toward the tip of the syringe barrel 8 one month after the assembly using water as a liquid for internal use, and the measurement was conducted using the universal resting instrument "Autograph" made by Shimadzu Corporation. The measurement results are shown in Table4 and Table 5. The measurement result for the piston 1 according to the present embodiment is shown in Fig.9(A) and the measurement result for the piston 1P according to the comparative example is shown in Fig.9(B), in which the horizontal axis is the stroke(mm) and the vertical axis is the sliding resistance value (N). The measurement was conducted assuming the cases of using the pistons 1 and 1P in prefilled syringes, and it is assumed that medicinal solutions are administered at medical institutes one month after the syringes were filled with the medicinal solutions.

**Table 4 sliding resistance value test, one month after assembly (present embodiment)**

| Sample | Average | Maximum value | Minimum value |
|---|---|---|---|
| 1 | 28.92N | 102.87N | 11.66N |
| 2 | 27.87N | 99.94N | 11.66N |
| 3 | 28.80N | 101.50N | 11.75N |

**Table 5 sliding resistance value test, one month after assembly (comparative example)**

| Sample | Average | Maximum value | Minimum value |
|---|---|---|---|
| 1 | 36.50N | 143.54N | 13.76N |
| 2 | 34.56N | 138.60N | 13.20N |
| 3 | 34.94N | 140.52N | 12.98N |

Similar to the test result shown in Fig.8(A),(B), as shown in the test result shown in

Fig. 9 (A), (B), the vicinity of the point, where the stroke is 0mm, corresponds to the beginning of the movement of each of the pistons 1 and 1P, where each measurement result shows the maximum sliding resistance value. And then the sliding resistance value decreases rapidly, and it shows the minimum sliding resistance value when each of the pistons 1 and 1P starts moving. Each of the pistons 1 and 1P was pushed in the syringe barrel 8 up to the predetermined position by moving toward the tip of the syringe barrel. The sliding resistance value one month after the assembly is larger than the sliding resistance value one day after the assembly.

As shown in Fig.9(A),(B) Table 4 and Table 5, both the average value and the maximum value of the sliding resistance in the piston 1 according to the present embodiment is more reduced than those in the piston 1P according to the comparative example even in the case of one month after the assembly. The minimum value of the sliding resistance in the piston 1 according to the present embodiment is also more reduced than the minimum value of the sliding resistance in the piston 1P according to the comparative example one month after the assembly. Therefore it is possible to preferably reduce the sliding resistance value of the piston according to the present embodiment which is inserted into the syringe barrel even if it is used in a prefilled syringe.

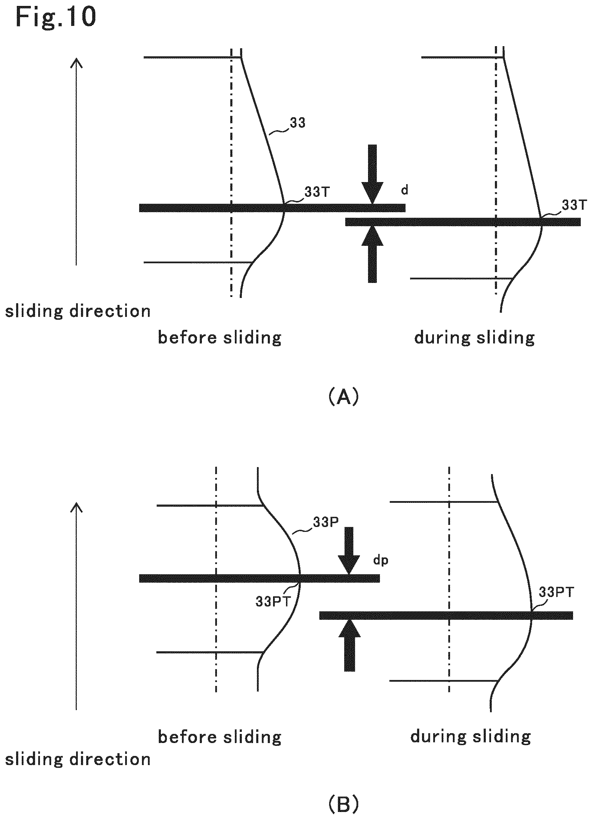

Fig.10(A) and Fig.10 (B) are schematic views for explaining the shapes of the second annular protrusions which can be considered as one of the reasons why the sliding resistance value is reduced in the present embodiment. In Fig.10(A), the vertex 33T of the second annular protrusion 33 according to the present embodiment is displaced backward in the sliding direction by the displacement "d" when the piston is inserted into the syringe barrel and slid in the direction shown by the arrow. On the other hand, in Fig.10(B), the vertex 33PT of the second annular protrusion 33P according to the comparative example is displaced backward in the sliding direction by the displacement "dp" when the piston is inserted into the syringe barrel and slid in the direction shown by the arrow.

As shown in the drawings, the displacement "d" is smaller than the displacement "dp". That is to say, the decreased sliding resistance value is considered to be contributed by a force for returning the piston 1 according to the present embodiment to the original position, namely the force for returning the shape of the second annular protrusion 33 to the original shape is smaller than the force for returning the piston 1P according to the comparative example to the original position, namely the force for returning the shape of the second annular protrusion 33P to the original shape.

If the height H of the second annular protrusion 33P according to the comparative example is set to be smaller the sliding resistance value becomes smaller, however it becomes difficult to keep a high sealing performance. On the other hand, it is possible to make the sliding resistance value smaller and keep a high sealing performance without making the height H of the annular protrusion 33 according to the present embodiment smaller.

In the piston 1 according to the present embodiment, the first annular protrusion 31 has a curved surface having an arc shaped cross-section in the axial direction, the curvature radius of the vertex 31T is R, and it is larger than the curvature radius 0.6R of the vertex 33T of the second annular protrusion 33 and the curvature radius 0.8R of the vertex 35T of the third annular protrusion 35. In this configuration, it is considered that the shape of the first annular protrusion 31 plays a role of keeping a high sealing performance mainly, and the shapes of the second annular protrusion 33 and the third annular protrusion 35 each play a role of reducing the sliding resistance value while keeping a high sealing performance.

In the present embodiment, three annular protrusions are formed and the maximum diameter part 33T of the second annular protrusion 33 is formed at a position which is shifted from the halfway (a position which is 1/2 of the axial length) of the second annular protrusion 33 toward the side of the bottom surface 4. However the number of the annular protrusions is not limited to 3, although it is possible to form two annular protrusions or four or more annular protrusions, it is preferable to form two or three annular protrusions. Although it is possible to form the maximum diameter part of any annular protrusion at a position which is shifted from the halfway (a position which is 1/2 of the axial length) of the annular protrusion toward the bottom surface side, it is preferable to form at least one annular protrusion having such a maximum diameter part at the second or latter position counting from the tip side of the piston.

Although the piston is formed by non-laminated rubber in the above embodiment, it is possible to use a piston (a plastic laminate piston) in which the surface contacting with a medicinal solution or the sliding surface is laminated by a plastic film, such as fluorocarbon resin, ultrahigh molecular weight polyethylene, polyethylene, polypropylene, polyester, nylon, etc. The circumference of a piston may be laminated with a fluorocarbon resin film from a perspective of a stability and a water repellent property of the wetted part of the piston. It is also possible to use (1) a piston which is not laminated with a fluorocarbon resin film, (2) a piston in which the circumference (the upper surface and the side surface) is laminated with a fluorocarbon resin film or (3) a piston in which the circumference (at least the wetted surface at the side of the upper surface) is laminated with a fluorocarbon resin film as a piston according to the present invention.

It is possible to appropriately select a fluorocarbon resin from PTFE (polytetrafluoroethylene), ETFE(ethylene-tetrafluoroethylene copolymer), PFE(perfluoro alkoxy alkane), PFA(perfluoro ethylene propene copolymer), PVDF(polyvinylidene difluoride), etc. or an alloy of such a fluorocarbon resin and other polymer.

In the above embodiment, although a piston used for a 100mL syringe is explained, the present invention is applicable to a piston for a larger volume syringe or a smaller volume syringe without being limited to this size of piston.

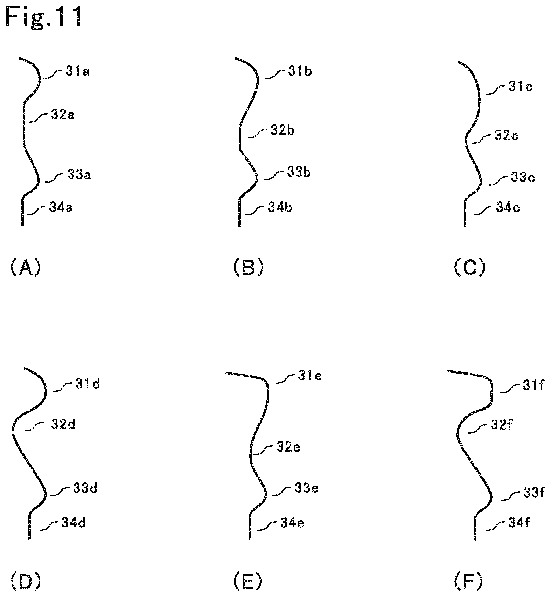

Next, other embodiments of the present invention will be explained referring to Fig. 11 with respect to the first annular protrusion, the first annular recess, the second annular protrusion and the second annular recess. As shown in Fig.11(A), it is also possible to keep a high sealing performance and reduce the sliding resistance value by forming a first annular protrusion 31a which has an arc shaped cross-section, a first annular recess 32a having a flat part and a second annular protrusion 33a. In this embodiment, the angle *θ* 2 of the second annular protrusion is set to be smaller than the angle *θ* 1 of the first annular protrusion similar to the embodiment shown in Fig.3(B). Although the angle *θ* 1 and the angle *θ* 2 are not shown in the drawing, they are the angles of the parts which are similar to those shown in Fig.3(B).

As shown in Fig.11(B), it is also possible to reduce the sliding resistance value while keeping a high sealing performance by making the axial length of a first annular protrusion 31b, which has an arc shaped cross-section partially, longer than the axial length of the first annular protrusion 31a in Fig.11 (A), making the flat part of a first annular recess 32b shorter than the flat part of the first annular recess 32a and forming a second annular protrusion 33b. The slope from the vertex of the first annular protrusion 31b toward the first annular recess 32b has a small tilt angle. In this embodiment, it is possible to set the angle *θ* 2 of the second annular protrusion to be equal to the angle *θ* 1 of the first annular protrusion, or larger or smaller than the angle *θ* 1 of the first annular protrusion.

As shown in Fig.11(C), it is also possible to reduce the sliding resistance value while keeping a high sealing performance by making the axial length of a first annular protrusion 31c having an arc shaped cross-section longer than the axial length of the first annular protrusion 31a in Fig. 11 (A), making the flat part of a first annular recess 32c shorter than the flat part of the first annular recess 32a in Fig.11 (A), and forming a second annular protrusion 33c. In this embodiment, it is possible to set the angle *θ* 2 of the second annular protrusion to be equal to the angle *θ* 1 of the first annular protrusion, or larger or smaller than the angle *θ* 1 of the first annular protrusion.

As shown in Fig.11 (D), it is also possible to reduce the sliding resistance value while keeping a high sealing performance by forming a first annular protrusion 31d, which has an arc shaped cross-section, a first annular recess 32d having no flat part and a second annular protrusion 33d. In this embodiment, the angle *θ* 2 of the second annular protrusion is set to be smaller than the angle *θ* 1 of the first annular protrusion.

As shown in Fig.11(E), it is also possible to reduce the sliding resistance value while keeping a high sealing performance by making the axial length of a first annular protrusion 31e longer than the axial length of the first annular protrusion 31a in Fig.11 (A) to have a flat part, making the flat part of a first annular recess 32e shorter than the flat part of the first annular recess 32a in Fig.11 (A) and forming a second annular protrusion 33e. There is a slope having a small tilt angle from the flat part of the first annular protrusion 31e toward the first annular recess 32e. In this embodiment, it is possible to set the angle *θ* 2 of the second annular protrusion to be equal to the angle *θ* 1 of the first annular protrusion, or larger or smaller than the angle *θ* 1 of the first annular protrusion.

As shown in Fig.11 (F), it is also possible to reduce the sliding resistance value while keeping a high sealing performance by making the axial length of a first annular protrusion 31f longer than the axial length of the first annular protrusion 31a in Fig.11 (A) to have a flat part, forming a first annular recess 32f having no flat part and forming a second annular protrusion 33f. In this embodiment, the angle *θ* 2 of the second annular protrusion is set to be smaller than the angle *θ* 1 of the first annular protrusion.

As shown in Fig.11(A) through (F), the outer diameter of each vertex part of the first annular protrusions 31a,31b,31c and 31d and each flat part of the first annular protrusions 31e and 31f is set to be equal to the outer diameter of each vertex part of the second annular protrusions 33a,33b,33c,33d,33e and 33f. Although the outer diameter of each bottom part of the first annular recesses 32a,32b,32c and 32e is set to be equal to the outer diameter of each bottom part of the second annular recesses 34a,34b,34c,34e, it is possible to set the outer diameter of the bottom parts of the first annular recesses 32d and 32f to be different from the outer diameter of the bottom parts of the second annular recesses 34d and 34f.

According to the above-described embodiment, it is possible to reduce the sliding resistance and prevent a liquid leakage. It is possible to keep a high sealing performance securely by preventing a piston from moving backward even at the time of sterilization, storage and transportation because it is considered that it increases the sliding pressure in the direction opposite to a sliding direction. It is also preferable to use a piston according to the present embodiment in a prefilled syringe in which a syringe barrel is filled with a medicinal solution and assembled to a syringe in advance. It is possible to keep a high sealing performance securely by preventing a piston from moving backward even at the time of sterilization, storage and transportation because it is considered that it increases the sliding pressure in the direction opposite to a sliding direction when the piston is used in a prefilled syringe.

### Explanation of References

- 1, 1P: piston
- 2, 2P: upper surface
- 3, 3P: side surface
- 4, 4P: bottom surface
- 5: screw hole
- 21: vertex
- 31,31P: first annular protrusion
- 32,32P: first annular recess
- 33,33P: second annular protrusion
- 34,34P: second annular recess
- 35,35P: third annular protrusion
- 36: third annular recess
- 31T,31PT: vertex
- 33T,33PT: vertex
- 35T,35PT: vertex
- 37: extended bottom surface
- 8: syringe barrel
- 81: cylinder part
- 82: flange part
- 83: inner surface

## Claims

1. A piston used by being inserted into a syringe barrel comprising an approximately cylindrical elastic body, wherein
the piston comprises an upper surface which is to be contacted with a liquid for internal use, a bottom surface with which a plunger rod is to be contacted and a side surface which is to be contacted with the inner surface of the syringe barrel, when the position is inserted into the syringe barrel,
the side surface includes plural annular protrusions in the axial direction,
the maximum diameter part of the annular protrusions has an outer diameter which is to be contacted with the inner surface of the syringe barrel when the piston is inserted into the syringe barrel, and
the maximum diameter part of at least one annular protrusion is formed at a position which is shifted from the halfway of the axial length of the annular protrusion toward the bottom surface side.

2. A piston according to Claim 1, the side surface has a first annular protrusion, an annular recess and a second annular protrusion in this order from the upper surface side in the axial direction,
the maximum diameter part of the first annular protrusion and the second annular protrusion has an outer diameter which is to be contacted with the inner surface of the syringe barrel when the piston is inserted into the syringe barrel, and
the maximum diameter part of the second annular protrusion is formed at a position which is shifted from the halfway of the axial length of the second annular protrusion toward the bottom surface side.

3. A piston according to Claim 1 or 2, the side surface has a first annular protrusion, an annular recess and a second annular protrusion in this order from the upper surface side in the axial direction,
the maximum diameter part of the first annular protrusion and the second annular protrusion has an outer diameter which is to be contacted with the inner surface of the syringe barrel when the piston is inserted into the syringe barrel, and
the curvature radius of the second annular protrusion is smaller than the curvature radius of the first annular protrusion.

4. A piston according to Claim 2 or 3, the tilt angle of the surface from the annular recess toward the maximum diameter part of the second annular protrusion relative to the annular recess is smaller than the tilt angle of the surface from the maximum diameter part toward the bottom surface side relative to the annular recess.

5. A piston according to either one of Claims 2 through 4, the contact area in which the second annular protrusion is to be contacted with the inner surface of the syringe barrel is smaller than the contact area in which the first annular protrusion is to be contacted with the inner surface of the syringe barrel when the piston is inserted into the syringe barrel.
